# EUROPEAN PATENT APPLICATION

(11) **EP 4 588 478 A1**
(43) Date of publication of application: **23.07.2025**
(21) Application number: 24749675.5
(22) Date of filing: 30.01.2024
(51) Int. Cl.: A61K 31/49, A61K 9/08, A61K 47/02, A61K 47/26, C07D 453/04, A61P 27/10

(54) **USE OF BENCYCLOQUIDIUM BROMIDE, ISOMER THEREOF AND DERIVATIVE THEREOF IN PREPARATION OF OPHTHALMIC FORMULATION**

(30) Priority: 01.02.2023 CN 202310050666
(71) Applicant: Beijing Yingu Medicine SCI Techno Co., Ltd., Beijing 100083 (CN)
(72) Inventor: YU, Wenzhan, Beijing 100083 (CN); PAN, Yuanyuan, Beijing 100083 (CN); LI, Yanyan, Beijing 100083 (CN); YUAN, Fengquan, Beijing 100083 (CN); LIU, Zihan, Beijing 100083 (CN); NI, Qingyuan, Beijing 100083 (CN); ZHANG, Ya, Beijing 100083 (CN)
(74) Representative: f & e patent
(86) International application number: PCT/CN2024/074678
(87) International publication number: WO 2024/160196

(57) **Abstract**

The present invention relates to the technical field of medicine, and in particular to a use of bencycloquidium bromide, an isomer thereof and a derivative thereof in the preparation of a drug for preventing or treating an ophthalmic disease. Moreover, the present invention provides an ophthalmic formulation using the bencycloquidium bromide, the isomer thereof and the derivative thereof as active ingredients. According to the present invention, by using the bencycloquidium bromide, the isomer thereof and the derivative thereof in eyes, it is discovered that the bencycloquidium bromide, the isomer thereof and the derivative thereof have an obvious inhibitory effect on changes in ocular axial length and diopter in the progression process of a myopic refractive error, and then it is discovered that the ophthalmic formulation using the bencycloquidium bromide, the isomer thereof and the derivative thereof as active ingredients has significant advantages in the prevention and treatment of ophthalmic diseases, especially in preventing myopia and slowing down the progression of myopia, etc.

## Description

### Cross-reference to related application

The present application claims the priority to Chinese Patent Application No. 202310050666.7, entitled "Use of bencycloquidium bromide, isomer thereof and derivative thereof in preparation of ophthalmic formulation", and filed on February 1, 2023, the entire disclosure of which is incorporated herein by reference in its entirety.

### Technical Field

The present application relates to the technical field of medicine, and in particular to a use of bencycloquidium bromide, an isomer thereof and a derivative thereof in the manufacture of a drug for preventing or treating an ophthalmic disease.

### Background Art

Myopia is a common eye disease. When the eye is in a relaxed state, parallel light rays enter the eye and focus in front of the retina, resulting in the inability to form a clear image on the retina. Myopia is generally divided into pseudomyopia, true myopia and mixed myopia. Pseudomyopia can be treated with drugs, acupuncture, ear acupuncture and physical therapy equipment, or through the manner for patients to strengthen their eye muscles by exercises, to relax muscles, relieve fatigue and restore vision to normal. True myopia generally requires corrective devices, surgery or drug.

In recent years, the incidence of myopia in China has been on the rise, and myopia among children and adolescents becomes more prevalent and occurs at younger ages. Currently, there are not many drugs available clinically for the prevention and treatment of myopia, mainly atropine eye drops with low-concentrations, whose controlling mechanism is still uncertain. Atropine eye drops with low-concentrations are easy to use, but they can cause adverse reactions such as photophobia, blurred near vision, and allergic conjunctivitis. Therefore, it is necessary to further research and develop drugs to treat or alleviate the progression of myopia and prevent myopia.

In view of this, the present application is proposed.

### Summary

In order to solve the above technical problems, the present application provides a use of bencycloquidium bromide, an isomer thereof and a derivative thereof in the manufacture of a drug for preventing or treating an ophthalmic disease. It has been found in the present application that bencycloquidium bromide, an isomer thereof and a derivative thereof have significant effects in preventing myopia and slowing down the progression of myopia, mydriasis and the like.

Specifically, the technical solutions of the present application are as follows:
In a first aspect, the present application provides a use of at least one of bencycloquidium bromide, an isomer thereof and a derivative thereof (which may be any one of bencycloquidium bromide, an isomer thereof and a derivative thereof, or a combination of any two of bencycloquidium bromide, an isomer thereof and a derivative thereof, or a combination of any three of bencycloquidium bromide, an isomer thereof and a derivative thereof) in the manufacture of a drug for preventing or treating an ophthalmic disease.

At present, bencycloquidium bromide, an isomer thereof and a derivative thereof are mainly used to improve the runny nose, nasal congestion, nasal itching and sneezing symptoms caused by allergic rhinitis. Bencycloquidium bromide, an isomer thereof and a derivative thereof are usually administered into the nasal cavity for the prevention and treatment of allergic rhinitis. In the present application, it is discovered for the first time that bencycloquidium bromide, an isomer thereof and a derivative thereof (nitrogen oxides, solvates, metabolites, pharmaceutically acceptable salts such as its organic salts or inorganic salts) can be effectively used to prepare drugs for preventing or treating ophthalmic diseases, and exhibit obvious advantages.

Specifically, in the present application, the bencycloquidium bromide isomer is selected from the structure shown in any one of Formula I, Formula II, Formula III or Formula IV:

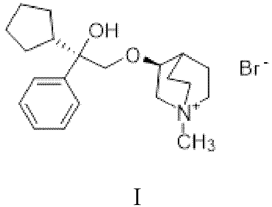

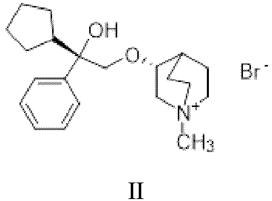

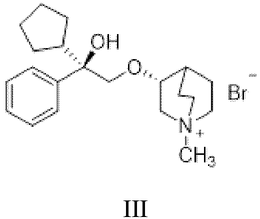

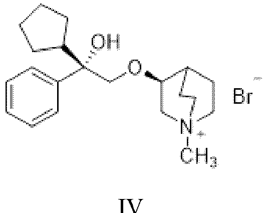

**In** the present application, the ophthalmic disease is selected from ciliary muscle paralysis, mydriasis, amblyopia, myopic refractive error or a combination thereof, in particular myopic refractive error. The present application can effectively prevent and delay the occurrence of myopic refractive error, protect the retina, and enhance the patient's ability to resist visual fatigue.

Furthermore, the myopic refractive error preferably comprises axial myopia or refractive myopia.

The present application has a therapeutic effect on different degrees of myopia types such as mild myopia, moderate myopia, and high myopia, different age groups of myopia types such as childhood myopia and adolescent myopia, and different moderating factors of myopia types such as true myopia and pseudomyopia.

According to the present application, by using at least one of the bencycloquidium bromide, the bencycloquidium bromide isomer and the bencycloquidium bromide derivative in eyes, and it is found that they have a significant inhibitory effect on the change of ocular axial length and diopter in the progression process of a myopic refractive error. That is, they can be effectively used for the prevention and treatment of ophthalmic diseases, especially myopic refractive error.

The present application provides a use of at least one of bencycloquidium bromide, an isomer thereof and a derivative thereof (which may be any one of bencycloquidium bromide, an isomer thereof and a derivative thereof, or a combination of any two of bencycloquidium bromide, an isomer thereof and a derivative thereof, or a combination of any three of bencycloquidium bromide, an isomer thereof and a derivative thereof) in the manufacture of a drug for preventing or treating an ophthalmic disease, wherein the drug is preferably an ophthalmic formulation.

In the present application, the dosage form of the ophthalmic formulation is not particularly limited, that is, any ophthalmic formulation prepared by using bencycloquidium bromide, an isomer thereof and a derivative thereof as pharmaceutically active ingredients and supplementing with a medium acceptable in the pharmaceutical field, for example, eye drops, eye ointments, eye creams, eye gels, eye films, eye pills, ocular inserts or lyophilized formulation. The lyophilized formulation can be used for ocular administration by dissolving in a dissolving diluent.

In a second aspect, the present application provides an ophthalmic formulation, wherein a pharmaceutically active ingredient of the ophthalmic formulation comprises at least one of bencycloquidium bromide, an isomer thereof and a derivative thereof (which can be a single active ingredient or a combination of multiple active ingredients), wherein, the bencycloquidium bromide isomer is preferably selected from the structure shown in any one of Formula **I,** Formula **II,** Formula **III** or Formula IV.

In some embodiments provided by the present application, it is preferred to prepare an ophthalmic formulation by using only one structure, namely, bencycloquidium bromide, an isomer thereof, or a derivative thereof as a single active ingredient.

In the present application, the bencycloquidium bromide, an isomer thereof and a derivative thereof are used as pharmaceutically active ingredients for preventing and treating myopia to prepare the ophthalmic formulation. On the one hand, the ophthalmic formulation is helpful for the rapid onset of the pharmaceutically active ingredients in the process of preventing and treating myopia; on the other hand, the ophthalmic formulation is helpful for reducing corresponding adverse reactions (systemic) while playing a role in preventing and treating myopia.

In a third aspect, the present application provides a specific ophthalmic formulation product, namely, eye drops. The eye drops provided by the present application contain a pharmaceutically active ingredient and water for injection; the a pharmaceutically active ingredient comprises at least one of bencycloquidium bromide, an isomer thereof and a derivative thereof (which may be a single active ingredient or a combination of multiple active ingredients); wherein the bencycloquidium bromide isomer is preferably selected from the structure shown in any one of Formula I, Formula II, Formula III or Formula IV.

The content of the pharmaceutically active ingredient in the eye drops is 0.01% to 0.5%, preferably 0.02% to 0.1%, more preferably 0.04% to 0.06%, and more preferably 0.05% by mass.

In the present application, an osmolality of the eye drops is preferably 250 to 350 mOsmol/kg, more preferably 280 to 320 mOsmol/kg.

In the present application, a pH value of the eye drops is preferably 5.5 to 9.0, more preferably 5.5 to 7.5, which has a better performance in improving the long-term stability of the eye drops compared to other pH value ranges.

Furthermore, in some preferred embodiments provided by the present application, when the osmolality of the eye drops is 280 to 320mOsmol/kg, the pH value is preferably 6.0 to 7.0. The above range of osmolality and the above range of pH value contribute to reduce eye irritation to the eye when the eye drops are used, and also contribute to the stability of the eye drops themselves.

In the prior art, the bencycloquidium bromide can be a non-liquid formulation, or a liquid formulation that cannot be directly applied to the eyes, and has a certain degree of irritation to the eyes. The eye drops provided by the present application are truly suitable for eye use by optimizing the formulation, and can play a good role in preventing myopia and slowing down the progression of myopia or dilating the pupil.

Specifically, the present application provides a plurality of preferred embodiments, wherein the eye drops of bencycloquidium bromide are prepared by adding a plurality of specific auxiliary ingredients or by a specific manufacture method.

In the present application, the eye drops may or may not be added with an osmotic pressure regulator according to specific situation. When an osmotic pressure regulator is added, the osmotic pressure regulator is preferably one or more selected from sodium chloride, mannitol, and sorbitol, especially sodium chloride, which contributes to reduce the irritation of the eye drops to the eyes.

In particular, when the osmotic pressure regulator is mannitol and/or sorbitol, a mass ratio of mannitol and/or sorbitol to the pharmaceutically active ingredient is preferably (0.5-2.0):(0.01-0.5), more preferably (0.5-2.0):(0.04-0.06); and a content of mannitol and/or sorbitol in the eye drops is preferably 0.5% to 2.0% by mass.

When the osmotic pressure regulator is sodium chloride, a mass ratio of sodium chloride to the pharmaceutically active ingredient is preferably (0.3-0.9):(0.01-0.5), more preferably (0.3-0.9):(0.04-0.5), more preferably (0.3-0.9):(0.04-0.06); and a content of sodium chloride in the eye drops is preferably 0.3% to 0.9% by mass.

In the present application, the eye drops may or may not be added with a thickener according to specific situation. When a thickener is added, the thickener is preferably one or more selected from glycerin, sodium hyaluronate, carbomer, polyvinyl alcohol, methylcellulose, hydroxypropyl methylcellulose, and sodium carboxymethyl cellulose, and more preferably polyvinyl alcohol, which have better performance than other thickeners in improving the ocular applicability of eye drops and extending the long-term stability of eye drops.

Furthermore, when the thickener is one or more selected from polyvinyl alcohol, sodium hyaluronate, carbomer, hydroxypropyl methylcellulose, hydroxyethyl cellulose, and sodium carboxymethyl cellulose, a content of the thickener in the eye drops is preferably 0.1% to 3.0%, more preferably 0.1% to 1.5% by mass.

When the thickener is polyvinyl alcohol, a content of polyvinyl alcohol in the eye drops is preferably 0.3% to 3.0%, more preferably 0.5% to 1.5%, more preferably 0.7 to 1.4%.

In the present application, preferably, a specific amount of thickener is added to the eye drops to help relieve eye fatigue and dryness symptoms caused by overuse.

Furthermore, in the present application, preferably, a mass ratio of the osmotic pressure regulator to the thickener is controlled to be (0.2-2.0):(0.1-3.0). More preferably, when the osmotic pressure regulator of the present application is sodium chloride, and the thickener is sodium hyaluronate or carbomer, a mass ratio of the osmotic pressure regulator to the thickener is preferably (0.3-0.9):(0.1-1.0), more preferably (0.3-0.9):(0.1-0.7).

When the osmotic pressure regulator of the present application is selected from mannitol and/or sorbitol, and the thickener is selected from polyvinyl alcohol, hydroxypropyl methylcellulose or sodium carboxymethyl cellulose, a mass ratio of the osmotic pressure regulator to the thickener is preferably (0.5-2.0):(0.1-1.0), more preferably (0.5-2.0):(0.3-0.7).

When the osmotic pressure regulator of the present application is sodium chloride, and the thickener is polyvinyl alcohol, a mass ratio of the osmotic pressure regulator to the thickener is preferably (0.3-0.9):(0.3-3.0), more preferably (0.3-0.9):(0.5-1.5), which is helpful for the long-term stability of the eye drops.

The specific osmotic pressure regulator and thickener controlled in the above ratio are helpful for increasing the residence time of the eye drops in the eyes, increasing the eye's moisturizing feeling, and improving comfort; and the eye drops will not be too viscous to cause eye discomfort or irritation.

In the present application, the eye drops may or may not be added with an antibacterial agent according to specific situation. When an antibacterial agent is added, the antibacterial agent is preferably one or more selected from benzyl alcohol, phenylethyl alcohol, benzoic acid, benzoate, benzalkonium chloride, benzalkonium bromide, benzethonium, phenoxyethanol, hydroxyphenyl esters, chlorobutanol, sorbic acid, and sorbate, preferably benzalkonium chloride, which is more helpful for reducing eye irritation of eye drops than other antibacterial agents.

Furthermore, when the antibacterial agent of the present application is benzalkonium chloride, a mass ratio of the benzalkonium chloride to the pharmaceutically active ingredient is preferably (0.01-0.05):(0.01-0.5), more preferably (0.01-0.05):0.05, and more preferably 0.02:0.05. A content of benzalkonium chloride in the eye drops is preferably 0.01% to 0.05%, more preferably 0.02% by mass.

In the present application, preferably a specific amount of antibacterial agent components is added to the eye drops, which ensures the sterility of the product while minimizing eye irritation, and thereby avoiding eye irritation or discomfort caused by the antibacterial agent.

The eye drops of the present application can be packaged in multiple doses or in single doses. No matter it is multi-dose or single-dose packaging, eye drops need to be controlled as sterile products. In another feasible but more costly specific embodiment provided by the present application, the eye drops can be prepared into single-dose aseptic packaging which does not use antibacterial agents and does not affect product efficacy.

In the present application, the eye drops may or may not be added with a pH adjuster according to specific situation. When a pH adjuster is added, the pH adjuster is preferably one or more selected from hydrochloric acid, sodium hydroxide, phosphate buffer, citric acid-sodium citrate buffer, citric acid-disodium hydrogen phosphate buffer, and boric acid-sodium hydroxide buffer, and is used to adjust the pH range of the eye drops.

Furthermore, the eye drops of the present application preferably use citric acid-sodium citrate buffer, boric acid-sodium hydroxide buffer or phosphate buffer as a pH regulator, which has better eye applicability.

The present application does not particularly limit the sources of the various auxiliary materials listed above, and conventional and commercially available auxiliary material products in the art can be used as raw materials for the eye drops of the present application for product manufacture.

In a fourth aspect, the present application provides a method for preparing the eye drops, comprising the step of dissolving a pharmaceutically active ingredient and the osmotic pressure regulator in water for injection or water for injection containing an antibacterial agent. In the present application, the pharmaceutically active ingredient and the osmotic pressure regulator are dissolved in water for injection at the same time, so that the pharmaceutically active ingredient can be better dispersed, which is helpful for reducing eye irritation.

Preferably, the manufacture method further comprises the step of swelling or dissolving the thickener in water for injection to obtain a thickener solution; the manufacture method comprises dissolving the pharmaceutically active ingredient and the osmotic pressure regulator in water for injection or water for injection containing an antibacterial agent, and then mixing the resultant with the thickener solution.

In the present application, the amount of water for injection used to swell or dissolve the thickener is preferably 5% to 15% of the total mass of the eye drops. In the present application, the thickener is swollen or dissolved in injection water alone, and then mixed with a solution containing a pharmaceutically active ingredient and an osmotic pressure regulator. This operation method can more evenly disperse the thickener into the eye drop product, allowing the thickener to completely swell or dissolve, thereby ensuring that the eye drop product has an appropriate consistency and improving the quality of the eye drop product.

In an alternative specific embodiment of the present application, the manufacture method comprises the following steps:
S1. when the antibacterial agent is a hydroxyphenyl ester, dissolving the antibacterial agent in water for injection at 60°C to 80°C to obtain solution 1; when the antibacterial agent is other than a hydroxyphenyl ester, dissolving the antibacterial agent in water for injection at 20°C to 60°C to obtain solution 1;
S2. swelling or dissolving the thickener in water for injection to obtain solution 2;
S3. dissolving the osmotic pressure regulator and bencycloquidium bromide in water for injection at room temperature to obtain solution 3;
S4. mixing solution 1, solution 2 and solution 3 evenly, cooling to about 40°C or less or to room temperature, and adjusting the pH value of the resultant to 5.5 to 9.0 with a pH adjuster, and adding water for injection to make up the full amount; and
S5. filtering the resultant through a 0.22 µm filter membrane or filter cartridge, bottling under sterile conditions to obtain eye drops of bencycloquidium bromide.

In another more preferred embodiment of the present application, the manufacture method comprises the following steps:
S1. when the antibacterial agent is a hydroxyphenyl ester, dissolving the antibacterial agent in water for injection at 60°C to 80°C to obtain solution 1; when the antibacterial agent is other than a hydroxyphenyl ester, dissolving the antibacterial agent in water for injection at 20°C to 60°C to obtain solution 1;
S2. dissolving the osmotic pressure regulator and bencycloquidium bromide in solution 1 to obtain solution 2;
S3. swelling or dissolving the thickener in water for injection to obtain solution 3;
S4. mixing solution 2 and solution 3 evenly, cooling to about 40°C or less or to room temperature, and adjusting the pH value of the resultant to 5.5 to 9.0 with a pH adjuster, and adding water for injection to make up the full amount; and
S5. filtering the resultant through a 0.22 µm filter membrane or filter cartridge, bottling under sterile conditions to obtain eye drops of bencycloquidium bromide.

In the above two specific embodiments, the pH value of the eye drops is preferably controlled at 5.5 to 7.5.

In a fifth aspect, the present application also provides the use of at least one of bencycloquidium bromide, an isomer thereof and a derivative thereof, or the ophthalmic formulation, or the eye drops, or the eye drops prepared by the manufacture method in preventing myopia and slowing down the progression of myopia or dilating the pupil.

### Beneficial effects:

The present application provides a use of at least one of bencycloquidium bromide, an isomer thereof and a derivative thereof in the manufacture of a drug for preventing or treating an ophthalmic disease, and specifically provides an eye drop with at least one of bencycloquidium bromide, an isomer thereof and a derivative thereof as an active ingredient. The eye drop can be effectively used for treating ophthalmic diseases. On the one hand, the eye drop has significant advantages in preventing myopia and slowing down the progression of myopia, and the like; on the other hand, the eye drop shows good stability in both long-term experiments and accelerated experiments, and after a 12-month long-term stability experiment or a 6-month accelerated experiment, the eye drop generally maintains the initial medicinal activity and safety, has no obvious irritation to the eyes, is not dry, has stable quality, and has excellent prospects for promotion and application.

### Brief Description of the Drawings

In order to more clearly illustrate the technical solutions in the present application or the prior art, the drawings required for use in the embodiments or the description of the prior art will be described below.
Figure 1 is a graph showing a change in ocular diopter over time after 4 weeks of the experiment in which the eye drops of bencycloquidium bromide prepared in experimental example 1 of the present application were applied to an animal model experiment of form-deprivation myopia.
Figure 2 is a comparison graph showing the change value of ocular diopter in the fourth week of the experiment in which the eye drops of bencycloquidium bromide prepared in experimental example 1 of the present application were applied to an animal model experiment of form-deprivation myopia.
Figure 3 is a graph showing a change in ocular axial length over time after 4 weeks of the experiment in which the eye drops of bencycloquidium bromide prepared in experimental example 1 of the present application were applied to an animal model experiment of form-deprivation myopia.
Figure 4 is a comparison graph showing the change value of ocular axial length in the fourth week of the experiment in which the eye drops of bencycloquidium bromide prepared in experimental example 1 of the present application were applied to an animal model experiment of form-deprivation myopia.

### Specific Modes for Carrying Out the Embodiments

The technical solutions provided by the present application are described in detail below in conjunction with the embodiments, but the embodiments should not be construed as limiting the protection scope of the present application. Unless otherwise specified, the experimental methods used in the examples are conventional methods; the materials and reagents used are all available from commercial sources.

### Example 1

The present Example provides an eye drop containing bencycloquidium bromide as an active ingredient, and having a specific prescription as follows:
Active ingredient: 0.05% of bencycloquidium bromide;
Osmotic pressure regulator: Sodium chloride, used to adjust an osmolality of eye drop to 300 to 320 mOsmol/kg;
Thickener: 1.0% of polyvinyl alcohol;
Antibacterial agent: 0.02% of benzalkonium chloride;
pH adjuster: phosphate buffer (NaH₂PO₄-Na₂HPO₄), used to adjust the pH of eye drop to 6.8;
Water for injection: make up to full amount.

The present Example also provides a method for preparing the above-mentioned eye drop, and the specific manufacture steps were as follows:
(1) according to the prescription ratio, the antibacterial agent was dissolved in water for injection at 30°C to obtain solution 1;
(2) according to the prescription ratio, the osmotic pressure regulator and bencycloquidium bromide were dissolved in solution 1 to obtain solution 2;
(3) according to the prescription ratio, the thickener was added to 10% of the prescription amount of water for injection, and stirred until the thickener is completely dissolved to obtain solution 3, wherein the temperature of the water for injection was 25°C;
(4) solution 2 and solution 3 were evenly mixed, cooled to room temperature, and the pH value of the resultant was adjusted with a pH adjuster and water for injection was added to make up the full amount; and
(5) the resultant was filtered through a 0.22 µm filter membrane or filter cartridge, and bottled under sterile conditions to obtain eye drops of bencycloquidium bromide.

### Example 2

The present Example provides an eye drop containing bencycloquidium bromide as an active ingredient, and having a specific prescription as follows:
Active ingredient: 0.05% of bencycloquidium bromide;
Osmotic pressure regulator: Sodium chloride, used to adjust an osmolality of eye drop to 280 to 300 mOsmol/kg;
Thickener: 1.4% of polyvinyl alcohol;
Antibacterial agent: 0.02% of benzalkonium chloride;
pH adjuster: phosphate buffer (NaH₂PO₄-Na₂HPO₄), used to adjust the pH of the eye drop to 6.5;
Water for injection: make up to full amount.

The present Example also provides a method for preparing the above-mentioned eye drop, and the specific manufacture steps were as follows:
(1) according to the prescription ratio, the antibacterial agent was dissolved in water for injection at 30°C to obtain solution 1;
(2) according to the prescription ratio, the osmotic pressure regulator and bencycloquidium bromide were dissolved in solution 1 to obtain solution 2;
(3) according to the prescription ratio, the thickener was dissolved in water for injection to obtain solution 3; and
(4) solution 2 and solution 3 were evenly mixed, cooled to room temperature, and the pH value of the resultant was adjusted with a pH adjuster, and water for injection was added to make up the full amount.

The resultant was filtered through a 0.22 µm filter membrane or filter cartridge, and bottled under sterile conditions to obtain eye drops of bencycloquidium bromide.

### Example 3

The present Example provides an eye drop containing bencycloquidium bromide as an active ingredient, and having a specific prescription as follows:
Active ingredient: 0.05% of bencycloquidium bromide;
Osmotic pressure regulator: mannitol, used to adjust the osmolality of the eye drop to 300 to 310 mOsmol/kg;
Thickener: 0.7% of polyvinyl alcohol;
Antibacterial agent: 0.08% of ethylparaben;
pH adjuster: phosphate buffer (NaH₂PO₄-Na₂HPO₄), used to adjust the pH of the eye drop to 6.0 to 6.5;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 60°C to obtain solution 1;
(2)according to the prescription ratio, the osmotic pressure regulator and bencycloquidium bromide were dissolved in solution 1 to obtain solution 2;
(3) according to the prescription ratio, the thickener was dissolved in water for injection to obtain solution 3;
(4) solution 2 and solution 3 were evenly mixed, cooled to room temperature, and the pH value of the resultant was adjusted with a pH adjuster, and water for injection was added to make up the full amount; and
(5) the resultant was filtered through a 0.22 µm filter membrane or filter cartridge, and bottled under sterile conditions to obtain eye drops of bencycloquidium bromide.

### Example 4

The present Example provides an eye drop containing bencycloquidium bromide as an active ingredient, and having specific prescription as follows:
Active ingredient: 0.05% of bencycloquidium bromide;
Osmotic pressure regulator: Sodium chloride, used to adjust an osmolality of eye drop to 300 to 310 mOsmol/kg;
Thickener: 0.1% of carbomer;
Antibacterial agent: 0.025% of benzalkonium chloride;
pH adjuster: boric acid-sodium hydroxide buffer, used to adjust the pH of the eye drop to 7.0 to 7.5;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 35°C to obtain solution 1;
(2) according to the prescription ratio, the osmotic pressure regulator and bencycloquidium bromide were dissolved in solution 1 to obtain solution 2;
(3) according to the prescription ratio, the thickener was dissolved in water for injection to obtain solution 3;
(4) solution 2 and solution 3 were evenly mixed, cooled to room temperature, and the pH value of the resultant was adjusted with a pH adjuster, and water for injection was added to make up the full amount; and
(5) the resultant was filtered through a 0.22 µm filter membrane or filter cartridge, and bottled under sterile conditions to obtain eye drops of bencycloquidium bromide.

### Example 5

The present Example provides an eye drop containing bencycloquidium bromide as an active ingredient, and having a specific prescription as follows:
Active ingredient: 0.01% of bencycloquidium bromide;
Osmotic pressure regulator: sorbitol, used to adjust the osmolality of the eye drop to 290 to 300 mOsmol/kg;
Thickener: 0.3% of sodium carboxymethyl cellulose;
Antibacterial agent: 0.3% of phenylethanol;
pH adjuster: citric acid-sodium citrate, used to adjust the pH of the eye drop to 6.5 to 7.0;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 35°C to obtain solution 1;
(2) according to the prescription ratio, the osmotic pressure regulator and bencycloquidium bromide were dissolved in solution 1 to obtain solution 2;
(3) according to the prescription ratio, the thickener was dissolved in water for injection to obtain solution 3;
(4) solution 2 and solution 3 were evenly mixed, cooled to room temperature, and the pH value of the resultant was adjusted with a pH adjuster, and water for injection was added to make up the full amount; and
(5) the resultant was filtered through a 0.22 µm filter membrane or filter cartridge, and bottled under sterile conditions to obtain eye drops of bencycloquidium bromide.

### Example 6

The present Example provides an eye drop with bencycloquidium bromide as an active ingredient, and having a specific prescription as follows:
Active ingredient: 0.1% of bencycloquidium bromide;
Osmotic pressure regulator: Sodium chloride, used to adjust an osmolality of eye drop to 290 to 300 mOsmol/kg;
Antibacterial agent: 0.05% of benzalkonium chloride;
pH adjuster: hydrochloric acid;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 45°C to obtain solution 1;
(2) according to the prescription ratio, the osmotic pressure regulator and bencycloquidium bromide were dissolved in solution 1 to obtain solution 2;
(3) solution 2 was cooled to about 40°C or less, the pH value was adjusted to 6.0 with a pH adjuster, and water for injection was added to make up the full amount and
(4) the resultant was filtered through a 0.22 µm filter membrane or filter cartridge, and bottled under sterile conditions to obtain an eye drop of bencycloquidium bromide.

### Example 7

The present Example provides an eye drop with bencycloquidium bromide isomer as an active ingredient, and having a specific prescription as follows:
Active ingredient: 0.05% of bencycloquidium bromide isomer (structure shown in formula IV);
Osmotic pressure regulator: sodium chloride, used to adjust an osmolality of eye drop to 290 to 300 mOsmol/kg;
pH adjuster: phosphate buffer (NaH₂PO₄-Na₂HPO₄), used to adjust the pH of the eye drop to 6.5 to 7.0;
Water for injection: make up to full amount.

The present Example also provides a method for preparing the above-mentioned eye drops, and the specific manufacture steps were as follows:
(1) according to the prescription ratio, the osmotic pressure regulator and bencycloquidium bromide isomer were dissolved in water for injection to obtain solution 1;
(2) after solution 1 has been cooled to room temperature, the pH value was adjusted with phosphate buffer (NaH₂PO₄-Na₂HPO₄), and water for injection was added to make up the full amount; and
(3) the resultant was filtered through a 0.22 µm filter membrane or filter cartridge, and bottled under sterile conditions to obtain an eye drop of bencycloquidium bromide isomer.

### Example 8

The present Example provides an eye drop containing bencycloquidium bromide as an active ingredient, and having a specific prescription as follows:
Active ingredient: 0.05% of bencycloquidium bromide;
Osmotic pressure regulator: Sodium chloride, used to adjust an osmolality of eye drop to 300 to 320 mOsmol/kg;
Antibacterial agent: 0.02% of benzalkonium chloride;
pH adjuster: phosphate buffer (NaH₂PO₄-Na₂HPO₄), used to adjust the pH of the eye drop to 6.8;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 30°C to obtain solution 1;
(2) according to the prescription ratio, the osmotic pressure regulator and bencycloquidium bromide were dissolved in solution 1 and cooled to room temperature, the pH value was adjusted with a pH adjuster, and water for injection was added to make up the full amount; and
(3) the resultant was filtered through a 0.22 µm filter membrane or filter cartridge, and bottled under sterile conditions to obtain eye drops of bencycloquidium bromide.

### Example 9

The present Example provides an eye drop containing bencycloquidium bromide as an active ingredient, and having a specific prescription as follows:
Active ingredient: 0.05% of bencycloquidium bromide;
Osmotic pressure regulator: Sodium chloride, used to adjust an osmolality of eye drop to 300 to 320 mOsmol/kg;
Thickener: 0.7% of hydroxyethylcellulose;
Antibacterial agent: 0.02% of benzalkonium chloride;
pH adjuster: phosphate buffer (NaH₂PO₄-Na₂HPO₄), used to adjust the pH of the eye drop to 6.8;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 30°C to obtain solution 1;
(2) according to the prescription ratio, the osmotic pressure regulator and bencycloquidium bromide were dissolved in solution 1 to obtain solution 2;
(3) according to the prescription ratio, the thickener was added to 10% of the prescription amount of water for injection at 25°C while stirring, and the resultant was kept stirring for 60 minutes to obtain solution 3;
(4) solution 2 and solution 3 were evenly mixed, cooled to room temperature, and the pH value of the resultant was adjusted with a pH adjuster, and water for injection was added to make up the full amount; and
(5) the resultant was filtered through a 0.22 µm filter membrane or filter cartridge, and bottled under sterile conditions to obtain eye drops of bencycloquidium bromide.

### Example 10

The present Example provides an eye drop containing bencycloquidium bromide as an active ingredient, and having a specific prescription as follows:
Active ingredient: 0.05% of bencycloquidium bromide;
Osmotic pressure regulator: sodium chloride, used to adjust an osmolality of eye drop to 300 to 320 mOsmol/kg;
Thickener: 1.0% of polyvinyl alcohol;
Antibacterial agent: 0.02% of benzalkonium chloride;
pH adjuster: phosphate buffer (NaH₂PO₄-Na₂HPO₄), used to adjust the pH of the eye drop to 8.0;
Water for injection: make up to full amount.

The present Example also provides a method for preparing the above-mentioned eye drops, and the specific manufacture steps were as follows:
(1) according to the prescription ratio, the antibacterial agent was dissolved in water for injection at 30°C to obtain solution 1;
(2) according to the prescription ratio, the osmotic pressure regulator and bencycloquidium bromide were dissolved in solution 1 to obtain solution 2;
(3) according to the prescription ratio, the thickener was added to 10% of the prescription amount of water for injection, and stirred until the thickener was completely dissolved to obtain solution 3, wherein the temperature of the water for injection was 25°C;
(4) solution 2 and solution 3 were evenly mixed, cooled to room temperature, and the pH value of the resultant was adjusted with a pH adjuster, and water for injection was added to make up the full amount; and
(5) the resultant was filtered through a 0.22 µm filter membrane or filter cartridge, and bottled under sterile conditions to obtain eye drops of bencycloquidium bromide.

### Experimental Example 1

In the present Experimental Example, the eye drops of bencycloquidium bromide prepared in Example 1 were used to perform a myopia model experiment on colorful guinea pigs, specifically as follows.

An animal model of form-deprivation myopia (FDM) was prepared by using the monocular deprived (MD) method, i.e., covering one eye with an opaque eye patch to cause form-deprivation.

Eighteen healthy guinea pigs that have been screened and met the experimental requirements were randomly divided into three groups, namely, a model control group, a positive control group, and a bencycloquidium bromide group. Models were established for each group of animals, and drugs were started on the first day of modeling. The model control group (FDM), positive control group (FDM + atropine), and bencycloquidium bromide group (FDM +the eye drops of bencycloquidium bromide) were given blank solvent, 0.05% atropine, and 0.05% eye drops of bencycloquidium bromide by instillation into the conjunctival sac, once a day, 20 µL/eye, for a total of four weeks. Among them, the component of the blank solvent is water for injection; the components of 0.05% atropine using water for injection as solvent comprises 0.05% of atropine, 1.0% of polyvinyl alcohol, and 0.02% of benzalkonium chloride; and the osmotic pressure regulator is sodium chloride, and the osmolality of the eye drops is adjusted to 300 to 320 mOsmol/kg, and the pH regulator is phosphate buffer (NaH₂PO₄-Na₂HPO₄), which is used to adjust the pH value of the eye drops to 6.8.

The detection indicators were as follows:
(1) Animals are generally observed clinically once a day.
(2) Animal body weight is measured upon receipt, on the 7^{th} and 14^{th} days of quarantine, and twice a week after modeling.
(3) The anterior chamber depth, lens thickness, vitreous cavity depth and ocular axial length were checked before modeling and once a week after administration.
(4) The diopter was checked before modeling and once a week after administration.

Before modeling, there were no significant differences in ocular diopter and axial length among the animals in each group. After four weeks of the experiment, the experiment results of each group of animals were as follows:
During the experiment, there was no significant difference in body weight among the groups of animals. Compared with the model control group, the change value of ocular axial length in the positive control group and bencycloquidium bromide group was significantly reduced after four weeks of administration. The bencycloquidium bromide can inhibit the process of axial lengthening of guinea pigs in myopia model by judging from the changes in ocular axial length.

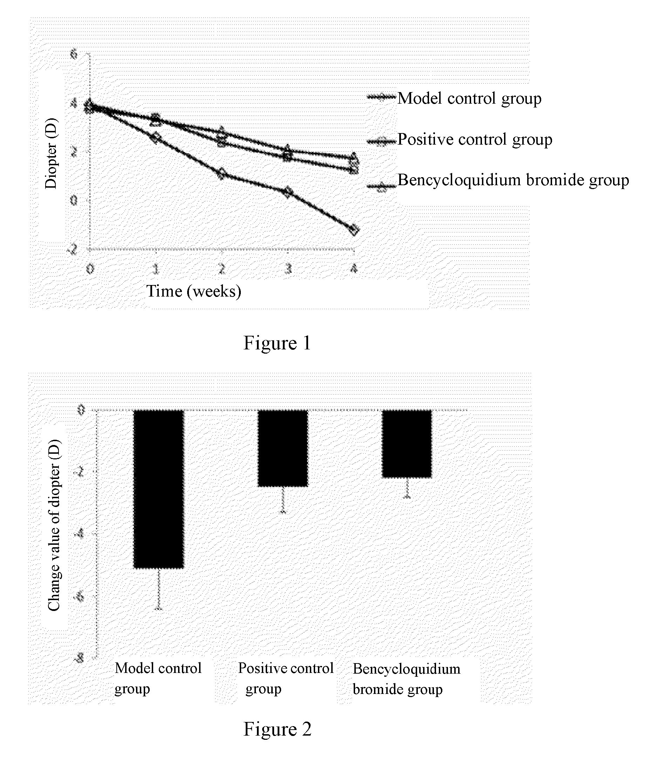

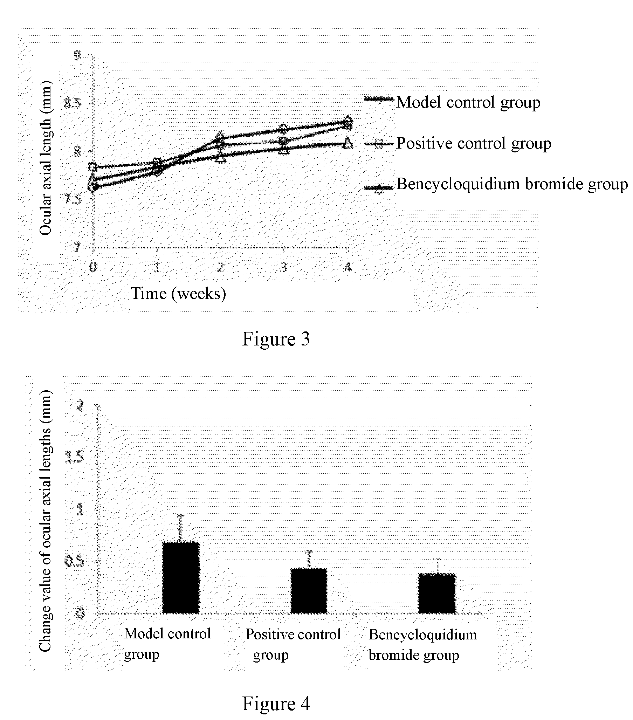

The experimental results of ocular diopter and axial length were shown in Figures 1-4.

Experiments have shown that by covering the eyes for four weeks to prepare a FDM myopia model of guinea pig, it can be seen that the diopter value has a significant downward trend, the ocular axial length is significantly prolonged, and there is an obvious trend of myopia model. Eye drops of 20 µL of 0.05% bencycloquidium bromide per day during the modeling period can significantly inhibit the trend of diopter decline and eye axial lengthening of guinea pigs in FDM model. Bencycloquidium bromide has a significant preventive effect on the guinea pigs in FDM myopia model.

The myopia model experiments were repeated with the eye drops obtained in other Examples, and basically the same conclusion was obtained, therefore the obvious prevention effects on myopia are shown.

### Experimental Example 2

In the present Experimental Example, the eye drops of bencycloquidium bromide prepared in Example 1, Example 3, Example 5, Example 8 and Example 9 are used to perform rabbit experiments, specifically as follows:
Twenty-five New Zealand rabbits, both male and female, weighing 2 to 2.5 kg, were randomly divided into five groups. The rabbits are administered using the method of comparing the left and right sides of the same body, with the test sample given to the left eye and an equal amount of blank given to the right eye. Experimental group 1 was given test sample 1 (eye drops provided in Example 8), experimental group 2 was given test sample 2 (eye drops provided in Example 9), experimental group 3 was given test sample 3 (eye drops provided in Example 1), experimental group 4 was given test sample 4 (eye drops provided in Example 3), and experimental group 5 was given test sample 5 (eye drops provided in Example 5), and the blank was water for injection. The drugs were administered twice a day, once in the morning and once in the afternoon, for consecutive 14 days, 20 µL/time/eye. The irritation reaction of the cornea, iris, conjunctiva and other eye tissues was observed by slit lamp before administration every day and 1h, 2h, 4h, 24h, 48h, and 72h after each administration.

It is found from the experiment that during the experiment, the conjunctiva of the left eyes of two rabbits in experimental group 1 is bright red and slightly edematous, the conjunctiva of the left eye of one rabbit in experimental group 2 is bright red, no obvious irritation reaction is shown in experimental group 3, the left eye of one rabbit of experimental group 4 has mild edema, and the conjunctiva of the left eye of one rabbit of experimental group 5 has slight bloodshot and slight edema. In the study of irritation to rabbit eye tissue, it is found that the eye drops containing polyvinyl alcohol as a thickener had the least irritation, while the eye drops without thickener (Example 8) had a stronger irritation to rabbit eye tissue.

### Experimental example 3

In the present Experimental example, the eye drops of bencycloquidium bromide prepared in Example 1, Example 8, Example 9 and Example 10 were used to perform a long-term stability experiment and an accelerated stability experiment. Among them, sample A is sourced from the eye drops provided in Example 9, sample B is sourced from the eye drops provided in Example 1, sample C is sourced from the eye drops provided in Example 8, and sample D is sourced from the eye drops provided in Example 10. The long-term stability experiments were performed under conditions of 25°C and RH 60%; and the accelerated stability experiments were performed under conditions of 40°C and RH 75%.

The results of the long-term stability experiments were shown in Table 1.

**Table 1: The results of the long-term stability experiments**

| Inspection project | | Character | pH value | Content (%) | Related substances (%) |
|---|---|---|---|---|---|
| Limit requirements | | It should be a colorless and transparent liquid | 6.0 to 8.0 | 90.0 to 110.0 | Total impurities must not exceed 2.0 |
| Samples | Time/ months | | | | |
| Sample A (Example 9) | 0 | Colorless and transparent liquid | 6.8 | 99.3 | 0.23 |
| | 3 | Colorless and transparent liquid | 6.8 | 99.3 | 0.34 |
| | 6 | Colorless and transparent liquid | 6.8 | 99.1 | 0.57 |
| | 9 | Colorless and transparent liquid | 6.8 | 98.8 | 0.73 |
| | 12 | Colorless and transparent liquid | 6.9 | 98.9 | 0.92 |
| Sample B (Example 1) | 0 | Colorless and transparent liquid | 6.8 | 99.2 | 0.22 |
| | 3 | Colorless and transparent liquid | 6.8 | 99.3 | 0.25 |
| | 6 | Colorless and transparent liquid | 6.8 | 99.2 | 0.32 |
| | 9 | Colorless and transparent liquid | 6.8 | 99.1 | 0.33 |
| | 12 | Colorless and | 6.8 | 99.0 | 0.41 |
| | | transparent liquid | | | |
| Sample C (Example 8) | 0 | Colorless and transparent liquid | 6.8 | 99.1 | 0.25 |
| | 3 | Colorless and transparent liquid | 6.8 | 99.0 | 0.37 |
| | 6 | Colorless and transparent liquid | 6.8 | 98.8 | 0.61 |
| | 9 | Colorless and transparent liquid | 6.9 | 98.6 | 0.86 |
| | 12 | Slightly yellow and transparent liquid | 6.8 | 98.4 | 1.02 |
| Limit requirements | | It should be a colorless and transparent liquid | 7.0 to 9.0 | 90.0 to 110.0 | Total impurities must not exceed 2.0 |
| Samples | Time/ months | | | | |
| Sample D (Example 10) | 0 | Colorless and transparent liquid | 8.0 | 99.1 | 0.24 |
| | 3 | Colorless and transparent liquid | 8.0 | 98.7 | 0.45 |
| | 6 | Colorless and transparent liquid | 8.0 | 97.8 | 0.55 |
| | 9 | Colorless and transparent liquid | 8.1 | 97.3 | 0.92 |
| | 12 | Slightly yellow and transparent liquid | 8.1 | 96.7 | 1.43 |

The results of the accelerated stability experiment were shown in Table 2.

**Table 2: The results of the accelerated stability experiment**

| Inspection project | | character | pH value | Content (%) | Related substances (%) |
|---|---|---|---|---|---|
| Limit requirements | | It should be a colorless and transparent liquid | 6.0 to 8.0 | 90.0 to 110.0 | Total impurities must not exceed 2.0 |
| Samples | Time/ months | | | | |
| Sample A (Example 9) | 0 | Colorless and transparent liquid | 6.8 | 99.3 | 0.23 |
| | 1 | Colorless and transparent liquid | 6.8 | 99.2 | 0.37 |
| | 2 | Colorless and transparent liquid | 6.8 | 98.9 | 0.52 |
| | 3 | Colorless and transparent liquid | 6.8 | 98.8 | 0.84 |
| | 6 | Slightly yellow and transparent liquid | 6.9 | 98.4 | 1.26 |
| Sample B (Example 1) | 0 | Colorless and transparent liquid | 6.8 | 99.2 | 0.22 |
| | 1 | Colorless and transparent liquid | 6.8 | 99.1 | 0.28 |
| | 2 | Colorless and transparent liquid | 6.8 | 99.1 | 0.38 |
| | 3 | Colorless and transparent liquid | 6.8 | 99.0 | 0.42 |
| | 6 | Colorless and transparent liquid | 6.8 | 98.9 | 0.55 |
| Sample C (Example 8) | 0 | Colorless and transparent liquid | 6.8 | 99.1 | 0.25 |
| | 1 | Colorless and transparent liquid | 6.8 | 98.8 | 0.41 |
| | 2 | Colorless and transparent liquid | 6.8 | 98.7 | 0.79 |
| | 3 | Slightly yellow and transparent liquid | 6.9 | 98.3 | 1.47 |
| | 6 | Slightly yellow and transparent liquid | 6.8 | 97.9 | 1.86 |
| Limit requirements | | It should be a Colorless and transparent liquid | 7.0 to 9.0 | 90.0 to 110.0 | Total impurities must not exceed 2.0 |
| Samples | Time/mont hs | | | | |
| Sample D (Example 10) | 0 | Colorless and transparent liquid | 8.0 | 99.1 | 0.24 |
| | 1 | Colorless and transparent liquid | 8.0 | 98.2 | 0.55 |
| | 2 | Colorless and transparent liquid | 8.1 | 97.5 | 0.91 |
| | 3 | Slightly yellow and transparent liquid | 8.1 | 95.8 | 1.58 |
| | 6 | Slightly yellow and transparent liquid | 8.2 | 94.6 | 2.31 |

The results of 12-month long-term stability experiments and 6-month accelerated stability experiments show that:
Sample B, which uses polyvinyl alcohol as the thickener, has no significant changes in content and related substances, and has the best stability;
The content of sample C without a thickener decreased significantly, and the related substances of sample C without a thickener increased significantly;
The content of sample A decreased slightly, and the related substances of sample A increased slightly;

In addition, when samples B and D with the same thickener were used and the eye drops were controlled at different pH values, the stability of sample B and the stability of sample D also shows differences. The content and related substances of sample B at pH 6.8 showed no significant changes, while the content of sample D at pH 8.0 decreased significantly, and the related substances of sample D increased significantly. The stability of the sample at pH 6.8 was better than that at pH 8.0.

### Experimental Example 4

In the present Experimental example, the eye drops of bencycloquidium bromide of Example 1, Example 8, Example 9, and Example 10 which have undergone 12-month of long-term stability experiments in Experimental Example 3 are used to perform rabbit experiments, specifically as follows.

Twenty New Zealand rabbits, both male and female, weighing 2 to 2.5 kg, were randomly divided into four groups. The rabbits are administered using the method of comparing the left and right sides of the same body, with the test sample given to the left eye and an equal amount of blank given to the right eye. Experimental group 1 was given test sample 1 (eye drops provided in Example 8), experimental group 2 was given test sample 2 (eye drops provided in Example 9), experimental group 3 was given test sample 3 (eye drops provided in Example 1), experimental group 4 was given test sample 4 (eye drops provided in Example 10), and the blank was water for injection. The drugs were administered twice a day, once in the morning and once in the afternoon, for consecutive 14 days, 20 µL/time/eye. The irritation reaction of the cornea, iris, conjunctiva and other eye tissues was observed by slit lamp before administration every day and 1h, 2h, 4h, 24h, 48h, and 72h after each administration.

It is found from experiment that during the experiment, the conjunctiva of the left eyes of four rabbits in experimental group 1 is bright red and slightly edematous; the conjunctiva of the left eye of one rabbit in experimental group 2 is bright red and slightly edematous, and the conjunctiva of the left eyes of two rabbits had slight bloodshot; no obvious irritation reaction is shown in experimental group 3; and the conjunctiva of the left eyes of two rabbits in experimental group 4 was bright red, and the conjunctiva of the left eye of 1 rabbit had slight bloodshot and slight edema.

The above-described Examples merely express several implementation methods of the present application, which are used to facilitate a specific and detailed understanding of the technical solution of the present application, but they should not be construed as limiting the scope of the application patent protection. It should be pointed out that, for those skilled in the art, several modifications and improvements can be made without departing from the concept of the present application, which all fall within the protection scope of the present application.

### Industrial applicability

The present application provides a use of bencycloquidium bromide, an isomer thereof and a derivative thereof in the manufacture of a drug for preventing or treating an ophthalmic disease. Moreover, the present application provides an ophthalmic formulation using bencycloquidium bromide, an isomer thereof and a derivative thereof as active ingredients. According to the present application, by using the bencycloquidium bromide, the isomer thereof and the derivative thereof in eyes, it is discovered that the bencycloquidium bromide, the isomer thereof and the derivative thereof have an obvious inhibitory effect on changes in ocular axial length and diopter in the progression process of a myopic refractive error, and then it is discovered that the ophthalmic formulation using the bencycloquidium bromide, the isomer thereof and the derivative thereof as active ingredients has significant advantages in the prevention and treatment of ophthalmic diseases, especially in preventing myopia and slowing down the progression of myopia, etc, and has good economic value and application prospects.

## Claims

1. Use of bencycloquidium bromide, an isomer thereof and a derivative thereof in the manufacture of a drug for preventing or treating an ophthalmic disease.

2. The use of claim 1, wherein the bencycloquidium bromide isomer is selected from the structure shown in any one of Formula I, Formula II, Formula III or Formula IV:

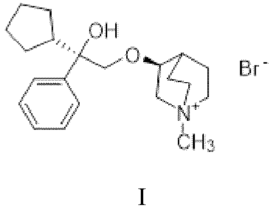

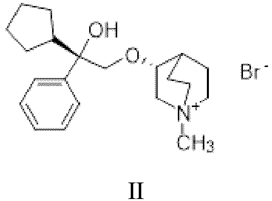

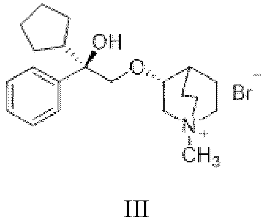

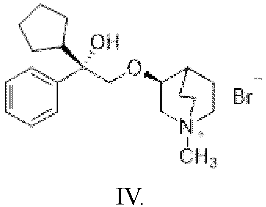

3. The use of claim 1 or 2, wherein the ophthalmic disease comprises myopic refractive error.

4. The use of any of claims 1 to 3, wherein the drug is an ophthalmic formulation;
preferably, the ophthalmic formulation comprises eye drops, eye ointments, eye creams, eye gels, eye films, eye pills, or ocular inserts.

5. An ophthalmic formulation, wherein a pharmaceutically active ingredient of the ophthalmic formulation comprises at least one of bencycloquidium bromide, an isomer thereof and a derivative thereof.

6. Eye drops, wherein the eye drops contain a pharmaceutically active ingredient and water for injection; the pharmaceutically active ingredient comprises at least one of bencycloquidium bromide, an isomer thereof and a derivative thereof; and a content of the pharmaceutically active ingredient in the eye drops is 0.01% to 0.5% by mass;
preferably, an osmolality of the eye drops is 250 to 350 mOsmol/kg; and/or a pH value of the eye drops is 5.5 to 9.0.

7. The eye drops of claim 6, wherein the eye drops contain at least one of an osmotic pressure regulator and a thickener;
preferably, the osmotic pressure regulator is one or more selected from sodium chloride, mannitol, and sorbitol; the thickener is one or more selected from glycerol, sodium hyaluronate, carbomer, polyvinyl alcohol, methyl cellulose, hydroxypropyl methylcellulose, hydroxyethyl cellulose, and sodium carboxymethyl cellulose;
more preferably, a mass ratio of the osmotic pressure regulator to the thickener is (0.2-2):(0.1-5.0).

8. The eye drops of claim 7, wherein the osmotic pressure regulator is sodium chloride, the thickener is polyvinyl alcohol, and a mass ratio of the sodium chloride to the polyvinyl alcohol is (0.3-0.9):(0.3-3.0).

9. The eye drops of any of claims 6 to 8, wherein the eye drops contain an antibacterial agent;
preferably, the antibacterial agent is one or more selected from benzyl alcohol, phenylethyl alcohol, benzoic acid, benzoate, benzalkonium chloride, benzalkonium bromide, benzethonium, phenoxyethanol, hydroxyphenyl esters, chlorobutanol, sorbic acid, and sorbate,
more preferably, the antibacterial agent is benzalkonium chloride; and a mass ratio of benzalkonium chloride to the pharmaceutically active ingredient is (0.01-0.05):(0.01-0.5).

10. A method for preparing the eye drops of any of claims 6 to 9, wherein the manufacture method comprises the step of dissolving a pharmaceutically active ingredient and an osmotic pressure regulator in water for injection or water for injection containing an antibacterial agent.

11. The manufacture method of claim 10, wherein the manufacture method further comprises the step of swelling or dissolving the thickener in water for injection to obtain a thickener solution;
preferably, the manufacture method comprises dissolving the pharmaceutically active ingredient and the osmotic pressure regulator in water for injection or water for injection containing an antibacterial agent, and then mixing a resultant with the thickener solution.

12. Use of bencycloquidium bromide, an isomer thereof and a derivative thereof, or the ophthalmic formulation of claim 5, or the eye drops of any of claims 6 to 9, or the eye drops prepared by the manufacture method of claim 10 or 11 in preventing myopia and slowing down the progression of myopia or dilating the pupil.
